# EUROPEAN PATENT APPLICATION

(11) **EP 4 778 478 A1**
(43) Date of publication of application: **22.07.2026**
(21) Application number: 25226750.5
(22) Date of filing: 23.12.2025
(51) Int. Cl.: A61B 18/02

(54) **CRYOABLATION PROBES WITH CRYOGEN FLOW DISRUPTORS**

(30) Priority: 17.01.2025 US 202519029794
(71) Applicant: Varian Medical Systems, Inc., Palo Alto, CA 94304 (US)
(72) Inventor: PHAMVU, Bryant, Austin, 78717 (US)
(74) Representative: Mathisen & Macara LLP

(57) **Abstract**

A cryoablation probe (200) includes a needle (202) forming an inner cavity. The needle includes at least one flow disruptor (206) positioned in the inner cavity. The cryoablation probe also includes a cryogen lumen (310) configured to deliver cryogen toward a distal end of the needle (204), wherein the at least one flow disruptor induces a turbulent flow of the cryogen in the inner cavity.

## Description

### FIELD

The present disclosure relates to cryoablation probes with cryogen flow disruptors. More particularly, the present disclosure relates to cryoablation probes with cryogen flow disruptors integrated into cryoablation needles to induce turbulent flow.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

It is desirable to perform cryoablation treatments quickly and efficiently to limit the amount of time that a patient is subjected to the medical treatment. In addition, reduced time and/or reduced amounts of cryogen and energy may reduce the cost of the treatment. It is desirable, therefore, to improve the rate of thermal energy transfer between the cryoablation probe and the targeted tissues. There exists a need, therefore, for improved cryoablation systems and probes that may allow for improved thermal energy transfer over existing or traditional systems and probes.

### SUMMARY

This section provides a general summary of the disclosure, and is not a comprehensive disclosure of its full scope or all of its features.

In various embodiments of the present disclosure, improved cryoablation probes are provided. The cryoablation probes or cryoprobes of the present disclosure may include flow disruptors that may disrupt a flow of cryogen in the needle of the cryoprobe. The flow disruptors may cause the cryogen to exhibit a turbulent flow inside the needle to improve thermal energy transfer between the cryogen inside the needle and the tissue positioned externally to the needle. This may improve efficiency and lower freezing cycle times over existing or traditional cryoprobes.

In some embodiments of the present disclosure, a cryoablation probe may include a needle forming an inner cavity. The needle may be an elongated tube that extends from a handle to a distal end or tip. The needle may be formed of a hollow tube to define an inner cavity therein. The needle may be made of various materials, for example, a stainless steel cylindrical tube. The needle may include measurement indicators positioned at predetermined axial locations along the exterior of the needle. The indicators may be laser etched or otherwise positioned on the needle. The needle may include at least one flow disruptor positioned in the inner cavity. The cryoablation probe may also include a cryogen lumen configured to deliver cryogen toward a distal end of the needle, wherein the at least one flow disruptor induces a turbulent flow of the cryogen in the inner cavity.

In one aspect, the at least one flow disruptor may be positioned on an inner surface of the needle.

In another aspect, the at least one flow disruptor may project inwardly from the needle in the inner cavity.

In another aspect, the at least one flow disruptor may be integrally formed in the needle. The flow disruptors may project radially inward from an inner surface of the needle. The flow disruptors may interrupt a flow of cryogen as it travels along the inner surface of the needle.

In another aspect, the at least one flow disruptor may include a plurality of flow disruptors, each flow disruptor of the plurality of flow disruptors projecting inwardly from the needle in the inner cavity.

In another aspect, the at least one flow disruptor may include a plurality of flow disruptors, each flow disruptor of the plurality of flow disruptors integrally formed in the needle.

In another aspect, the at least one flow disruptor may include a helical shape. In other examples, the flow disruptors may be in the form of dimples or other projections that project inward into the inner cavity of the needle.

In another aspect, the at least one flow disruptor may include a plurality of flow disruptors, each flow disruptor of the plurality of flow disruptors positioned at a different axial location along the needle.

The flow disruptors may have a suitable size, shape, length, and configuration such that a turbulent flow is induced within the needle. The flow disruptors may have a linear configuration. The flow disruptors may have a curved or parabolic cross-sectional shape. In another aspect, the at least one flow disruptor may include a crimp formed in the needle.

In another aspect, the at least one flow disruptor may include a rib on an inner surface of the needle. The flow disruptors may be positioned on an inner surface of the needle. The flow disruptors may be positioned at various angles relative to the longitudinal axis of the needle. In other examples, flow disruptors may be formed as ribs positioned in planes orthogonal to the longitudinal axis of the needle.

In another aspect, the flow disruptors may have a curved shape that extends along discrete portions of the needle. For example, the at least one flow disruptor may include a curved helical shape having a predetermined pitch and a predetermined length. The flow disruptors may be positioned along a double helix path such that flow disruptors are positioned in pairs on opposite circumferential sides of the needle. The flow disruptor may have a continuous helical shape that wraps around a circumference of the inner surface of the needle.

The height of the flow disruptors may have any suitable values and may depend on an overall diameter of the needle. In another aspect, a radial height of the at least one flow disruptor is in a range of about 0.12 mm to about 0.20 mm.

The cryogen lumen may be an elongated tubular member that extends in an inner cavity formed by the needle. The cryogen lumen may be concentrically positioned inside the inner cavity along the longitudinal axis of the needle. In use, the cryogen may travel inside the cryogen lumen to a position toward the tip of the needle. The cryogen may then travel back inside the needle along a return pathway between an outer surface of the cryogen lumen and an inner surface of the needle. In another aspect, the cryogen lumen may have an uneven outer surface. For example, the cryogen lumen may include one or more second flow disruptors positioned along an external surface of the cryogen lumen. The second flow disruptors may project radially outward from the cryogen lumen. The second flow disruptors may comprise ribs that project radially outward toward the inner surface of the needle in the inner cavity.

In another aspect, the cryogen lumen may include a corrugated outer surface. In another example, the second flow disruptor on the cryogen lumen may have a curved helical shape.

In some embodiments of the present disclosure, a cryoablation apparatus may include a cryoablation probe with flow disruptor that is coupled to a cryo-treatment console. The cryo-treatment console may include a cryo-controller, a cryogen delivery apparatus, and a cryogen source.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryo-treatment apparatus in accordance with some embodiments of the present disclosure.
FIG. 2 is a isometric view of an example cryoprobe needle in accordance with some embodiments of the present disclosure.
FIG. 3 is a side view of the cryoprobe of FIG. 2 showing internal elements of the cryoprobe in accordance with some embodiments of the present disclosure.
FIG. 4 is cross-sectional view of the cryoprobe needle of FIG. 2 cut along a plane as indicated.
FIG. 5 is a side view of another example cryoprobe illustrating a cryogen lumen with an uneven outer surface in accordance with some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In some embodiments of the present disclosure, a cryoprobe is provided that includes at least one flow disruptor positioned in the inner cavity of the needle. The flow disruptor may cause cryogen flowing in the needle to have a turbulent flow. The turbulent flow of the cryogen may cause an improved or increased thermal energy transfer to occur relative to existing or traditional cryoprobes. The improved thermal energy transfer may result in faster ice formation at the target tissue. The faster ice formation may result in shorter overall freezing cycles during cryoablation procedures.

In various embodiments of the present disclosure, the flow disruptors may project inwardly from the needle of the cryoprobe in a return path of the cryogen. The flow of the cryogen over the flow disruptors may cause turbulent flow in the return path.

In various embodiments described below, the cryo-based anesthesia apparatus may include a Joule-Thompson cryoprobe or a liquid Nitrogen cryoprobe to achieve the desired target temperatures. While some of the embodiments below may be described as using the Joule-Thompson effect to achieve cryoablation temperatures, it should be appreciated that aspects of the present disclosure may used with other cryoprobe types that may produce iceballs at target tissues using other cooling methods such as liquid Nitrogen or others.

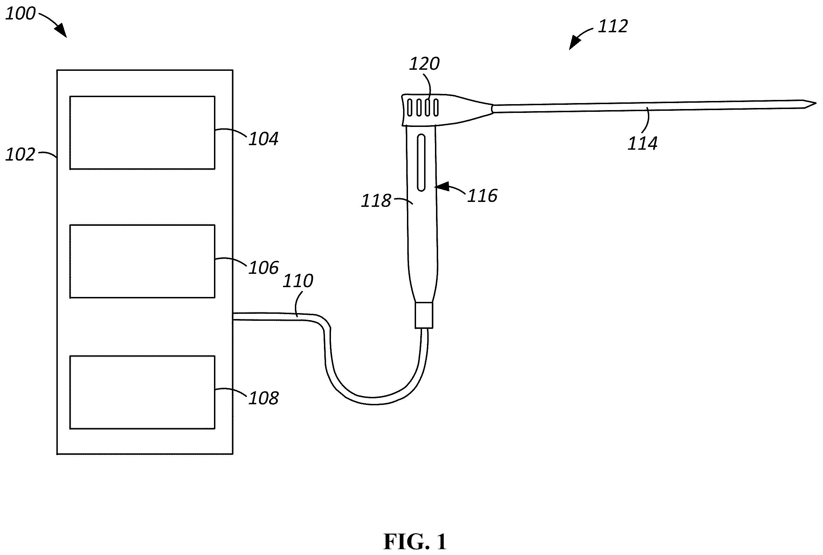

Referring now to FIG. 1, an example cryo-treatment apparatus 100 is shown. The cryo-treatment apparatus 100 may include a cryo-treatment console 102, and a cryoprobe assembly 112. The cryo-treatment console 102 may include a cryo-treatment control 104, a cryogen delivery apparatus 106 and a cryogen source 108. The cryo-treatment control 104 may include a computing device or other controller that can be used to control delivery of a cryogen (e.g., Argon, Helium, Nitrogen, or the like) from the cryogen source 108 to the cryoprobe assembly 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe assembly 112. Upon the initiation of a freezing cycle, the cryo-control 104 may cause the cryogen to be moved through a cryogen flow path that includes a cryogen supply line from the cryogen source through the cryogen line 110 to the cryoprobe 114. The cryogen may then flow back to the cryogen source via a cryogen return line from the cryoprobe 114 back to the cryogen source 108.

The cryogen line 110 may be a flexible tube or other conduit that may include multiple lumens to allow the cryogen to flow in a supply direction to the cryoprobe 114 and separately in a return direction away from the cryoprobe 114. The cryogen line 110 may of sufficient length to allow the console 102 to be positioned near the patient in a treatment room and to allow the patient to be moved into and out of an imaging device. In some examples, the cryogen line may be at least about 12 feet in length. In other examples, the cryogen line 110 may have other lengths.

The cryogen line 110 fluidly couples the cryogen source 108 to the cryoprobe 114. The cryoprobe 114 may be a needle or other elongated member that is configured to be inserted into patient tissue and be positioned at or near the target tissue during treatment. The cryoprobe 114 may include a cylindrical needle having an outer diameter in a range of about 1 mm to about 4 mm. The cryoprobe 114 may also include a handle 116. The handle 116 may be configured with a first portion 118 and a second portion 120. The first portion 118 may be substantially aligned with the cryogen line 110 and the second portion 120 may be offset at an angle relative to the first portion 118. The offset angle between the first portion 118 and the second portion 119 may be about 90 degrees to define a right angle handle. In other example, the first portion 118 and the second portion 120 may be offset at different angles.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 114. This may allow an operator to touch or otherwise manipulate the cryoprobe 114 during treatment.

While not shown, more than one cryoprobe assembly 112 may be coupled to the console 102. Multiple cryoprobe assemblies 112 may be used during a single cryo-procedure in combination. The console 102 may be configured to deliver cryogen to the multiple cryoprobe assemblies 112. The cryoprobes 114 of each cryoprobe assembly 112 may be similar to reach other or may be different to produce iceballs of different sizes and shapes so as to subject the target tissue to the cryo-treatment zone with a lowered temperature.

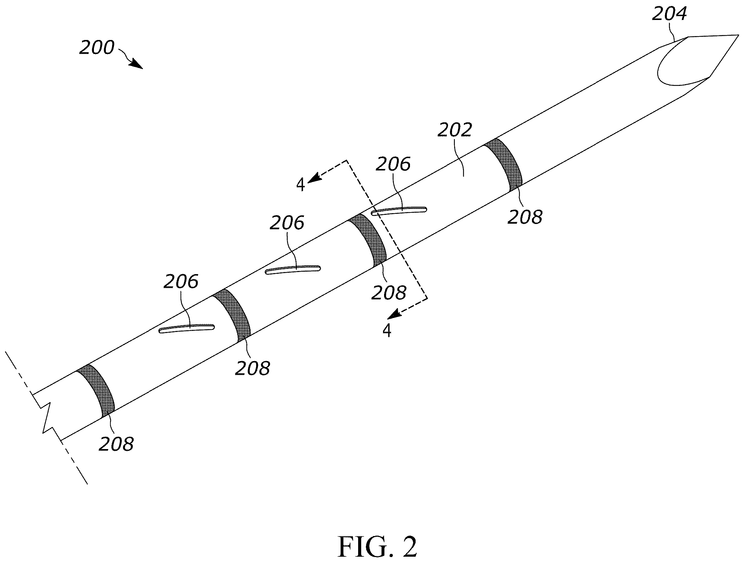

Referring now to FIGs. 2, 3 and 4, an example cryoprobe 200 is illustrated. In this example, the cryoprobe 200 may include a needle 202. The needle 202 may be an elongated tube that extends from a handle (not shown) to a distal end or tip 204. The needle may be formed of a hollow tube to define an inner cavity therein. Cryogen may be supplied to the needle 202 to lower the temperature of the needle 202 after it has been positioned at or near a target tissue. The needle 202 may be made of various materials. In some examples, the needle 202 may be formed of a stainless steel cylindrical tube. In other examples, other materials may be used. The needle 202 may include measurement indicators 208 that may be positioned at predetermined axial locations along the exterior of the needle 202. The indicators 208 may be used to determine a depth of insertion or withdrawal of the needle 202 when an operator is performing a cryoablation procedure. The indicators 208 may be laser etched or otherwise positioned on the needle 202.

The needle 202 may include one or more flow disruptors 206. In this example, the flow disruptors 206 may be formed in the needle 202. The flow disruptors 206 may be formed by crimping the needle 202 to have indentations at various positions along an axial or longitudinal direction of the needle. The flow disruptors may be visible on an exterior of the needle 202 since portions of the material of the needle 202 may be deformed to project radially inward toward a central axis of the needle 202.

In the example shown, the flow disruptors 206 may have a curved helical shape and may have a predetermined length. The shape, depth, and/or length of the flow disruptors may be determined through experimentation, simulation, or the like to produce a turbulent flow of the cryogen in the needle 202. As the cryogen flows over the inner surface of the needle 202, the cryogen flow will encounter the projections of the flow disruptors 206 to cause turbulent flow.

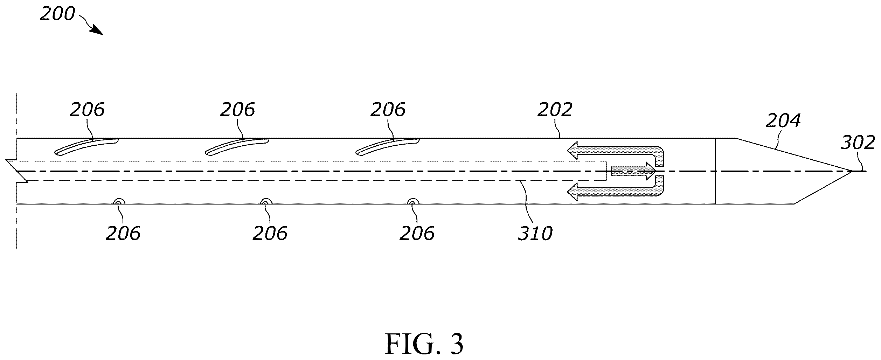

The cryogen may be provided to the cryoprobe 200 and flow through a cryogen lumen 310 (FIG. 3) in a direction toward the tip 204. The cryogen lumen 310 may be an elongated tubular member that extends in the inner cavity formed by needle 202. The cryogen lumen 310 may be concentrically positioned inside the inner cavity along axis 302 of the needle 202. The cryogen may travel inside the cryogen lumen 310 to a position toward the tip 204 where the cryogen may rapidly expand and drastically lower in temperature due to the Joule-Thompson effect. The cryogen may then travel back inside the needle 202 in a return pathway between an outer surface of the cryogen lumen 310 and an inner surface of the needle 202. As the cryogen returns in a direction away from the tip 204, the cryogen may encounter the flow disruptors 206.

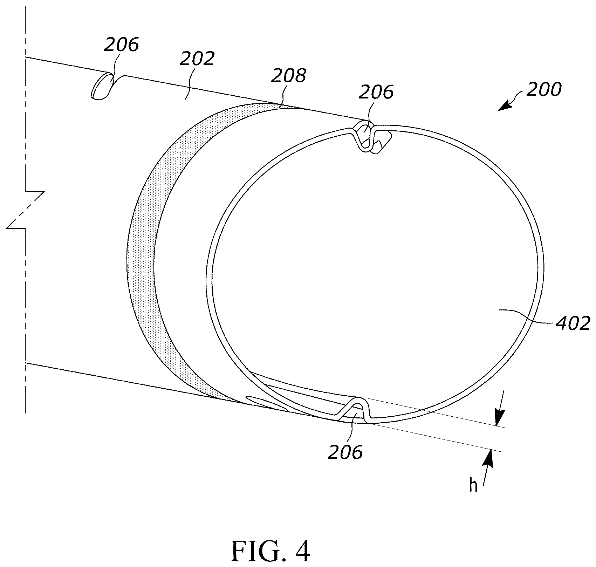

As shown in FIG. 4, for example, the flow disruptors 206 may project radially inward from an inner surface of the needle 202. The flow disruptors 206 may interrupt a flow of cryogen as it travels along the inner surface 402 of the needle 202. The flow disruptors 206 may have a suitable size, shape, length, and configuration such that a turbulent flow is induced within the needle 202. The turbulent flow of the cryogen may improve and increase the rate and amount of thermal transfer that occurs between the cryogen inside the needle 202 and the target tissue positioned externally to the needle 202. This, in turn, may increase a rate of formation of ice on the outside of the needle 202 during a cryoablation treatment.

As shown, the flow disruptors 206 may have a curved shape that extends along discrete portions of the needle 202. In this example, the flow disruptors 206 have a curved helical shape and may be positioned at regular intervals along the needle 202. The flow disruptors 206 may be positioned along a double helix path such that flow disruptors 206 are positioned in pairs on opposite circumferential sides of the needle 202. In the example shown, the flow disruptors 206 may have a predetermined height h that is measured orthogonally from the inner surface 402 of the needle to the apex or further radially inward point of the flow disruptor. In some examples, the height h may be in a range of about 0.12 mm to about 0.20 mm. In other examples, the height h may have other values and may depend on an overall diameter of the needle 202. The flow disruptors 206 may have a curved or parabolic cross-sectional shape.

In some examples, the flow disruptors 206 may be integrally formed in the needle 202. For purposes of this disclosure, integrally formed means that the flow disruptors flow are created as an unremovable part of the needle 202 and are created without adding additional material or components to the needle 202. In some examples, the integrally formed flow disruptors 206 may be formed by permanently deforming the original shape or profile of the needle 202. In other examples, the flow disruptors 206 may be integrally formed by molding, sintering, casting, stamping or the like.

In other examples, flow disruptors 206 may have shapes different from that shown above. In other examples, the flow disruptors 206 may be ribs that may be linear or curved. Such ribs may be positioned on an inner surface of the needle 202 and be positioned at various angles relative to the axis 302. In still other examples, flow disruptors 206 may be formed as ribs positioned in planes orthogonal to the axis 302.

In other examples, the flow disruptors 206 may be continuous rather than have discrete lengths along the needle 202. In such examples, the flow disruptors 206 may have a continuous helical shape (such as in the shape of an internal thread) that wraps around a circumference of the inner surface of the needle 202. In yet other examples, the flow disruptors may be in the form of dimples or other projections that project inward into the inner cavity of the needle 202.

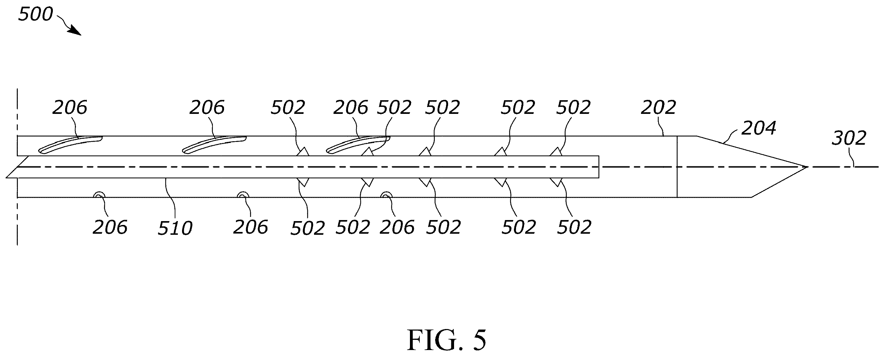

Referring now to FIG. 5, another example cryoprobe 500 is illustrated. The needle 202 in this example is similar to the needles previously described with respect to cryoprobe 200. The needle 202 may include flow disruptors 206 positioned along the needle that project inward into the inner cavity. In this example, the cryogen lumen 510 may be positioned in the inner cavity and may include one or more second flow disruptors 502 positioned along an external surface of the cryogen lumen 510. The second flow disruptors 502 may project radially outward from the cryogen lumen 510. In this example, the second flow disruptors 502 are shown as ribs that project radially outward toward the inner surface of the needle 202 in the inner cavity. The second flow disruptors 502 may also induce a turbulent flow of the cryogen that is flowing away from the tip 204.

In other examples, the cryogen lumen 510 may include a corrugated outer surface that may act as the second flow disruptor. The second flow disruptor on the cryogen lumen 510 may have a curved helical shape similar to flow disruptors 206 in still other examples.

The following is a list of non-limiting illustrative embodiments disclosed herein:
Illustrative embodiment 1: A cryoablation probe comprising: a needle forming an inner cavity, the needle comprising at least one flow disruptor positioned in the inner cavity; and a cryogen lumen configured to deliver cryogen toward a distal end of the needle; wherein the at least one flow disruptor induces a turbulent flow of the cryogen in the inner cavity.
Illustrative embodiment 2: The cryoablation probe of illustrative embodiment 1, wherein the at least one flow disruptor is positioned on an inner surface of the needle.
Illustrative embodiment 3: The cryoablation probe of any of illustrative embodiments 1 or 2, wherein the at least one flow disruptor projects inwardly from the needle in the inner cavity.
Illustrative embodiment 4: The cryoablation probe of any of illustrative embodiments 1 to 3, wherein the at least one flow disruptor is integrally formed in the needle.
Illustrative embodiment 5: The cryoablation probe of any of illustrative embodiments 1 to 4, wherein the at least one flow disruptor comprises a plurality of flow disruptors, each flow disruptor of the plurality of flow disruptors projecting inwardly from the needle in the inner cavity.
Illustrative embodiment 6: The cryoablation probe of any of illustrative embodiments 1 to 5, wherein the at least one flow disruptor comprises a plurality of flow disruptors, each flow disruptor of the plurality of flow disruptors integrally formed in the needle.
Illustrative embodiment 7: The cryoablation probe of any of illustrative embodiments 1 to 6, wherein the at least one flow disruptor comprises a helical shape.
Illustrative embodiment 8: The cryoablation probe of any of illustrative embodiments 1 to 7, wherein the at least one flow disruptor comprises a plurality of flow disruptors, each flow disruptor of the plurality of flow disruptors positioned at a different axial location along the needle.
Illustrative embodiment 9: The cryoablation probe of any of illustrative embodiments 1 to 8, wherein the at least one flow disruptor comprises a crimp formed in the needle.
Illustrative embodiment 10:The cryoablation probe of any of illustrative embodiments 1 to 9, wherein the at least one flow disruptor comprises a rib on an inner surface of the needle.
Illustrative embodiment 11:The cryoablation probe of any of illustrative embodiments 1 to 10, wherein the at least one flow disruptor comprises a curved helical shape having a predetermined pitch and a predetermined length.
Illustrative embodiment 12:The cryoablation probe of any of illustrative embodiments 1 to 11, wherein a radial height of the at least one flow disruptor is in a range of about 0.12 mm to about 0.20 mm.
Illustrative embodiment 13:The cryoablation probe of any of illustrative embodiments 1 to 12, wherein the cryogen lumen comprises an uneven outer surface.
Illustrative embodiment 14:The cryoablation probe of any of illustrative embodiments 1 to 13, wherein the cryogen lumen comprises a corrugated outer surface.
Illustrative embodiment 15:A cryoablation apparatus comprising the cryoablation probe of any of illustrative embodiments 1 to 14 coupled to a cryo-treatment console, the cryo-treatment console comprising a cryo-controller, a cryogen delivery apparatus, and a cryogen source.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoablation probe (114, 200, 500) comprising:
a needle (202) forming an inner cavity, the needle comprising at least one flow disruptor (206) positioned in the inner cavity; and
a cryogen lumen (310, 510) configured to deliver cryogen toward a distal end (204) of the needle;
wherein the at least one flow disruptor (206) induces a turbulent flow of the cryogen in the inner cavity.

2. The cryoablation probe of claim 1, wherein the at least one flow disruptor (206) is positioned on an inner surface (402) of the needle.

3. The cryoablation probe of claim 1 or 2, wherein the at least one flow disruptor (206) projects inwardly from the needle (202) in the inner cavity.

4. The cryoablation probe of claim 1, 2 or 3, wherein the at least one flow disruptor (206) is integrally formed in the needle (202).

5. The cryoablation probe of any one of the preceding claims, wherein the at least one flow disruptor (206) comprises a plurality of flow disruptors (206), each flow disruptor of the plurality of flow disruptors projecting inwardly from the needle (202) in the inner cavity.

6. The cryoablation probe of any one of the preceding claims, wherein the at least one flow disruptor comprises a plurality of flow disruptors (206), each flow disruptor of the plurality of flow disruptors integrally formed in the needle (202).

7. The cryoablation probe of any one of the preceding claims, wherein the at least one flow disruptor (206) comprises a helical shape.

8. The cryoablation probe of any one of the preceding claims, wherein the at least one flow disruptor comprises a plurality of flow disruptors, each flow disruptor (206) of the plurality of flow disruptors (206) positioned at a different axial location along the needle (202).

9. The cryoablation probe of any one of the preceding claims, wherein the at least one flow disruptor (206) comprises a crimp formed in the needle (202).

10. The cryoablation probe of any one of claims 1 to 8, wherein the at least one flow disruptor comprises (206) a rib on an inner surface (402) of the needle.

11. The cryoablation probe of any one of the preceding claims, wherein the at least one flow disruptor comprises a curved helical shape having a predetermined pitch and a predetermined length.

12. The cryoablation probe of any one of the preceding claims, wherein a radial height (h) of the at least one flow disruptor is in a range of about 0.12 mm to about 0.20 mm.

13. The cryoablation probe of any one of the preceding claims, wherein the cryogen lumen (510) comprises an uneven outer surface, and/or the cryogen lumen (510) comprises a corrugated outer surface.

14. The cryoablation probe of any one of the preceding claims, wherein the cryogen lumen (510) includes one or more second flow disruptors (502) positioned along an external surface of the cryogen lumen (510), optionally the second flow disruptors (502) project radially outward from the cryogen lumen (510), optionally the second flow disruptors (502) are ribs that project radially outward from the cryogen lumen (510) toward the inner surface (402) of the needle (202) in the inner cavity.

15. A cryoablation apparatus comprising the cryoablation probe (114,200) of claim 1 coupled to a cryo-treatment console (102), the cryo-treatment console comprising a cryo-controller (104), a cryogen delivery apparatus (106), and a cryogen source (108).
